# EUROPEAN PATENT SPECIFICATION

(11) **EP 2 626 087 B1**
(45) Date of publication and mention of the grant of the patent: **17.08.2016**
(21) Application number: 11830691.9
(22) Date of filing: 05.10.2011
(51) Int. Cl.: A61L 24/10

(54) **TISSUE ADHESIVE FILM AND METHOD FOR PRODUCING SAME**
GEWEBEHAFTFILM UND VERFAHREN ZU SEINER HERSTELLUNG
FILM D'ADHÉSIF TISSULAIRE ET PROCÉDÉ DE PRODUCTION DE CELUI-CI

(30) Priority: 05.10.2010 JP 2010225360
(43) Date of publication of application: 14.08.2013
(73) Proprietor: National Institute for Materials Science, Tsukuba-shi, Ibaraki 305-0047 (JP)
(72) Inventor: TAGUCHI Tetsushi, Tsukuba-shi Ibaraki 305-0047 (JP)
(74) Representative: V.O.
(86) International application number: PCT/JP2011/072962
(87) International publication number: WO 2012/046759

(56) References cited:
- EP-A1- 0 018 093
- EP-A1- 1 043 034
- WO-A1-2009/069727
- WO-A1-2010/026782
- JP-A- 7 163 860
- JP-A- 9 103 479
- JP-A- 11 239 609
- JP-A- 2007 182 407
- JP-A- 2008 125 916
- JP-A- 2008 284 256
- JP-A- 2009 515 620
- US-A1- 2005 282 748
- TETSUSHI TAGUCHI ET AL.: 'Soshiki Secchakuzai Kaihatsu no Genjo' ARTIFICIAL BLOOD vol. 14, no. 4, 25 May 2007, pages 118 - 123, XP008169591

## Description

### TECHNICAL FIELD

The present invention relates to a tissue adhesive film and a method for producing the same.

### BACKGROUND ART

A tissue adhesive film means a polymer film which is able to adhere to biological tissues such as blood vessels, skin, and the like (hereunder, referred to as tissue), in an operation such as cardiovascular surgery. With use of this film, it is possible to prevent blood leaking and such risks so as to improve the safety of the operation.

At present, there are three major types of tissue adhesive films as follows.

The first adhesive is a cyanoacrylate based tissue adhesive film. This polymer film has a problem in that the biocompatibility is low, although the adhesive strength is high.

The second adhesive is a biopolymer-aldehyde based tissue adhesive film. This polymer film also has a problem in that the biocompatibility is low, although the adhesive strength is high.

The third adhesive is a fibrin based tissue adhesive film. This polymer film has an opposite problem in that the adhesive strength is low, although the biocompatibility is high.

In this way, it has been so far a problem of this technology that there is no tissue adhesive film satisfying both excellent properties of the adhesive strength and the biocompatibility.

In recent years, regarding the fibrin based tissue adhesive film, it has been elucidated that a tissue adhesive comprising human serum albumin (hereunder, referred to as HSA) and a cross-linking agent has high adhesive strength (Non-Patent Document 1).

HSA is a serum protein made from blood preparations. It is a globular protein having a diameter of about 10 nm with a molecular weight of 69,000. Moreover, this is a negatively-charged acidic protein. In addition, disuccinimidyl tartarate (hereunder, referred to as DST) has been used as the cross-linking agent.

However, since any product using a blood preparation is classified as a medicinal product, considerable efforts are required for the approval and clearance. Moreover, once it is approved as a medicinal product, the usage record has to be kept continually for 20 years after the approval. This requires considerable efforts, which is a problem.

For this reason, it has been considered to use gelatin as a non-blood preparation instead of HSA. For example, JPH09103479 has disclosed a medical material prepared by crosslinking gelatin with succinimidized poly-L-glutamic acid. Moreover, JP2008125916A relating to a tissue adhesive film has disclosed a tissue adhesive film made from gelatin or collagen. However, they have a problem in that the adhesive strength is not enough.

In addiction, JP2006523113A relating to a tissue adhesive formulation has disclosed a tissue adhesive formulation which comprises a mixture of a synthetic and/or cross-linkable material in a particulate form and a particulate material. However, this tissue adhesive formulation also has a problem in that the adhesive strength is not enough.

Furthermore, a paper relating to gelatin in which an alkyl group has been introduced on the side chain, has been reported (Non-Patent Document 2).

EP0018093A1 relates to a two part gelatin tissue adhesive kit with citric acid or other cross-linking agents. EP1043034A1 relates to human serum albumin based tissue adhesives with genipin as cross-linker. US2005/282748A1 discloses tissue adhesives based on polymers such as collagen comprising an amino group crosslinked with a bifunctional linker.

However, solving the above-mentioned problems has yet to be achieved.

### PRIOR ART: NON-PATENT DOCUMENTS

Non-Patent Document 1: J. Bioact. Compact. Polym., 24, 546-559 (2009)
Non-Patent Document 2: Li-Huei Lin, Keng-Ming Chen, COLLOIDS AND SURFACES A, 272, 2006, 8-14
Non-Patent Document 3: Li-Huei Lin, Keng-Ming Chen, Chee-Chan Wang, Journal of Applied Polymer Science, 105, 2007, 3371-3377

### DISCLOSURE OF INVENTION

### PROBLEMS TO BE SOLVED BY THE INVENTION

The present invention addresses the problem of providing a tissue adhesive film having high adhesive strength and high biocompatibility and a method for producing the same.

### MEANS TO SOLVE THE PROBLEMS

The inventors of the present invention have discovered that a tissue adhesive film having high adhesive strength and high biocompatibility and a method for producing the same can be provided by using hydrophobically-modified gelatin which has an amino group and a hydrophobic group as defined by the claims on the side chain thereof, and has a molecular weight within a range of 50,000 or higher and 100,000 or lower, instead of HSA. This has led to the completion of the present invention.

The present invention has the following structures.

According to a first aspect, the present invention provides a tissue adhesive film as defined in claim 1.

In the tissue adhesive film of the present invention, the hydrophobically-modified gelatin may be a polymer in which two or more amino acids are linearly linked and a part of Lys residues in the polymer is substituted with the hydrophobic group.

In the tissue adhesive film of the present invention, the gelatin may be any one type or a combination of two or more types selected from the group consisting of natural gelatin derived from human, porcine, bovine, or fish species, or genetically modified gelatin thereof.

The tissue adhesive film of the present invention may be the above-mentioned tissue adhesive film in which the cross-linking reagent is any one type or a combination of two or more types selected from the group consisting of tartaric acid, citric acid, malic acid, glutamic acid, aspartic acid, oxalacetic acid, cis-aconitic acid, 2-ketoglutaric acid, polytartaric acid, polycitric acid, polymalic acid, polyglutamic acid, and polyaspartic acid.

According to a second aspect, the present invention provides a method for producing a tissue adhesive film as defined in claim 5.

In the method for producing a tissue adhesive film of the present invention, a step of adding an organic molecule that has a hydrophobic group, to a solution in which gelatin has been dissolved, under the presence of an amine, so as to synthesize the hydrophobically-modified gelatin by partially substituting amino groups on the side chain of the gelatin with the hydrophobic group, may be performed prior to the step (1).

In the method for producing a tissue adhesive film of the present invention, the fluorine based solvent may be hexafluoroisopropanol.

### EFFECT OF THE INVENTION

The tissue adhesive film of the present invention is a tissue adhesive film comprising an integration of gelatin and cross-linking reagents. The gelatin which constitutes the tissue adhesive film of the present invention is gelatin which is provided with an amino group on the side chain thereof and has a molecular weight of 50,000 or higher and 100,000 or lower, and the cross-linking reagent has two or more active ester groups or anhydrides within a single molecule. For this reason, the film can be stably used. Furthermore, the amino groups of the gelatin constituting the tissue adhesive film can be mutually cross-linked by the active ester groups or the acid anhydride of the cross-linking reagent. Therefore, a chemically firm attachment can be created. Moreover, the adhesive strength to the tissue can be enhanced through the amino groups on the side chain of the gelatin. Furthermore, since gelatin is used for the tissue adhesive film of the present invention, the film can be readily decomposed by an enzyme (collagenase) in the course of wound healing, by which the biocompatibility can be enhanced.

In the tissue adhesive film of the present invention, a part or all of the gelatin is hydrophobically-modified gelatin which is provided with a hydrophobic group on the side chain thereof. Therefore, if such hydrophobically-modified gelatin is used, a physically firm attachment with the tissue can be created by having the hydrophobic group piercing (anchored) into the tissue. Therefore, the adhesive strength to the tissue can be enhanced.

In the tissue adhesive film of the present invention, the hydrophobically-modified gelatin may be a polymer in which two or more amino acids are linearly linked and a part of Lys residues in the polymer is substituted with the hydrophobic group.

In the tissue adhesive film of the present invention, the hydrophobic group any one type or a combination of two or more types selected from the group consisting of a cholesteryl group, an oleyl group, an isostearyl group, a stearyl group, an isopalmityl group, a myristyl group, a lauryl group, a capric group, a pelargol group, a caprylic group, a caproyl group, an α-linolenyl group, a stearidonyl group, an eicosapentaenoyl group, and a docosahexaenyl group.

In the tissue adhesive film of the present invention, the gelatin may be any one type or a combination of two or more types selected from the group consisting of natural gelatin derived from human, porcine, bovine, or fish species, or genetically modified gelatin thereof.

In the tissue adhesive film of the present invention, the cross-linking reagent may be any one type or a combination of two or more types selected from the group consisting of tartaric acid, citric acid, malic acid, glutamic acid, aspartic acid, oxalacetic acid, cis-aconitic acid, 2-ketoglutaric acid, polytartaric acid, polycitric acid, polymalic acid, polyglutamic acid, and polyaspartic acid.

The method for producing a tissue adhesive film of the present invention comprises: (1) a step of dispersing hydrophobically-modified gelatin in a fluorine based solvent, and thereafter mixing it with a cross-linking reagent which has two or more active ester groups or anhydrides within a single molecule; and (2) a step of volatilizing the fluorine based solvent so as to obtain a film. Thus, if the fluorine based solvent is volatilized on a flat surface, the gelatin and the cross-linking reagent can be readily integrated while having them cross-linked with each other. Therefore, a tissue adhesive film having high adhesive strength and high biocompatibility can be readily produced.

In the method for producing a tissue adhesive film of the present invention, a step of adding an organic molecule that has a hydrophobic group, to a solution in which gelatin has been dissolved, under the presence of an amine, so as to synthesize the hydrophobically-modified gelatin by partially substituting amino groups on the side chain of the gelatin with the hydrophobic group, may be performed prior to the step (1).

In the method for producing a tissue adhesive film of the present invention, the fluorine based solvent may be hexafluoroisopropanol.

### BRIEF DESCRIPTION OF THE DRAWINGS

FIG. 1 is a cross-sectional schematic diagram illustrating an example of the tissue adhesive film of the present invention.
FIG. 2 is a schematic diagram illustrating an example of adhesion with use of the tissue adhesive film of the present invention.
FIG. 3 is a schematic diagram illustrating an example of the method for producing a tissue adhesive film of the present invention.

### EMBODIMENTS FOR CARRYING OUT THE INVENTION

### (Embodiments of the present invention)

Hereunder is a description of a tissue adhesive film and a method for producing the same, which are the embodiments of the present invention, with reference to the appended drawings.

### <Tissue adhesive film>

First, the tissue adhesive film serving as an embodiment of the present invention is described.

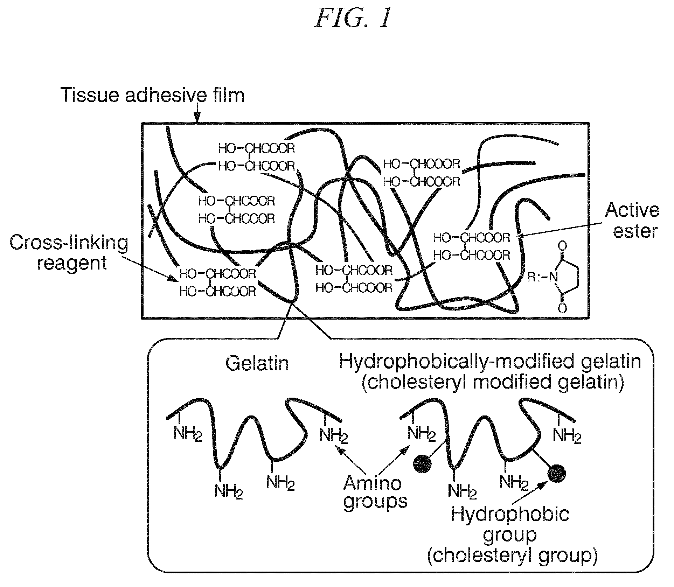

FIG. 1 is a cross-sectional schematic diagram illustrating an example of the tissue adhesive film serving as an embodiment of the present invention. As shown in FIG. 1, the tissue adhesive film is roughly composed of an integration of gelatin (unmodified gelatin which is not hydrophobically modified), hydrophobically-modified gelatin, and cross-linking reagents.

The tissue adhesive film may be composed of hydrophobically-modified gelatin and cross-linking reagents only, or may also be composed of gelatin and cross-linking reagents only.

For example, the tissue adhesive film is a film having an approximately rectangular shape in a planar view. One surface and the other surface of the film are made flat respectively. The size is not specifically limited. The film may have side lengths of 8 mm and a thickness of 100 µm, for example.

However, the shape of the tissue adhesive film is not limited to this shape and may be an approximately circular shape in a planar view.

For adhering a biological tissue (hereunder, abbreviated as a tissue) by using the tissue adhesive film, water is necessary to cause a cross-linking reaction between the gelatin or the hydrophobically-modified gelatin and the cross-linking reagent. However, because the biological tissue usually contains water, the tissue adhesive film absorbs water when the tissue adhesive film is pasted onto the tissue. Thus, the tissue can be adhered thereto.

The hydrophobically-modified gelatin comprises a main chain consisting of gelatin, and an amino group and the hydrophobic group on the side chain thereof. Because the gelatin structure is used for the tissue adhesive film of the present invention, the adhesive film can be readily decomposed by an enzyme within a living body, if applied to the living body. Therefore, the biocompatibility can be enhanced.

The hydrophobically-modified gelatin may be a polymer in which two or more amino acids are linearly linked and a part of Lys residues in the polymer is substituted with the hydrophobic group.

The gelatin can be exemplified by natural gelatin derived from human, porcine, bovine, or fish species, or genetically modified gelatin thereof. Any one type or a combination of two or more types of these gelatins may be used.

The molecular weights of the gelatin and the hydrophobically-modified gelatin are preferably 50,000 or higher and 100,000 or lower, more preferably 60,000 or higher and 100,000 or lower, and yet more preferably 70,000 or higher and 100,000 or lower. The adhesive strength can be improved by setting the molecular weight of the hydrophobically-modified gelatin within this range.

If the molecular weights of the gelatin and the hydrophobically-modified gelatin are lower than 50,000, the strength as a film is so low that the film would easily collapse. Therefore, such molecular weights are not preferable.

Lys is one of the protein-constituting α-amino acids, and is an essential amino acid. It is an amino acid having an ε-amino group on the side chain.

A part of the amino groups of Lys can be readily substituted with a hydrophobic group by a known method. Amino groups of the gelatin are partially substituted with a hydrophobic group.

The hydrophobic group in the hydrophobically-modified gelatin is anchored to the tissue when the hydrophobically-modified gelatin is subjected to the hydrolysis reaction. This makes it possible to firmly fix the hydrophobically-modified gelatin to the tissue. By so doing, the hydrophobically-modified gelatin can be adhered to the tissue by a physically firm attachment. Therefore, the adhesive strength when the tissue adhesive film of the present invention is in use, can be improved.

If the hydrophobic group is soluble with water, the hydrophobicity is low, which makes it difficult to stick the hydrophobic group to the tissue. Therefore, the hydrophobically-modified gelatin cannot be firmly fixed to the tissue. On the other hand, if the hydrophobic group is insoluble with water, the hydrophobic property is displayed, which makes it difficult to anchor the hydrophobic group to the tissue. Therefore, the hydrophobically-modified gelatin cannot be firmly fixed to the tissue.

The hydrophobic group can be exemplified by a cholesteryl group represented by the following formula (1), an oleyl group, an isostearyl group, a stearyl group, an isopalmityl group, a myristyl group, a lauryl group, a capric group, a pelargol group, a caprylic group, a caproyl group, an α-linolenyl group, a stearidonyl group, an eicosapentaenoyl group, and a docosahexaenyl group. In the tissue adhesive film of the present invention, the hydrophobic group can be any one type or a combination of two or more types selected from the group consisting of these.

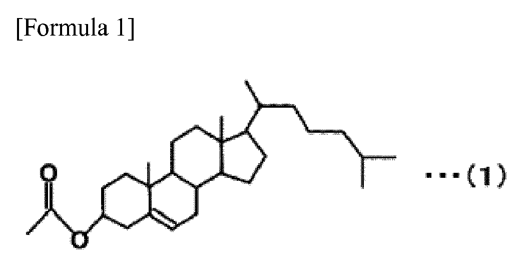

The cross-linking reagent comprises an organic molecule that has two or more active ester groups or anhydrides within a single molecule. By having two or more active ester groups or anhydrides within a single molecule, these cross-linking reagents are able to react with and bind to two amino groups of the gelatin or the hydrophobically-modified gelatin. Thus, a firm adhesive construct can be formed by cross-linking two or more amino groups of the gelatin or the hydrophobically-modified gelatin.

The cross-linking reagent can be exemplified by any one type or a combination of two or more types selected from the group consisting of tartaric acid, citric acid, malic acid, glutamic acid, aspartic acid, oxalacetic acid, cis-aconitic acid, 2-ketoglutaric acid, polytartaric acid, polycitric acid, polymalic acid, polyglutamic acid, and polyaspartic acid, wherein two or more active ester groups are held within a single molecule of the above-mentioned molecules, or an acid anhydride.

The active ester group is preferably any one type or a combination of two or more types ofN-hydroxysuccinimidyl or N-hydroxysulfosuccinimidyl groups. This is because succinimide is a succinic acid derivative existing in a metabolic pathway *in vivo*, and has been actually used for an FDA sanctioned tissue adhesive (sealant).

More specifically, the cross-linking reagent can be exemplified by disuccinimidyl tartrate represented by the following formula (2).

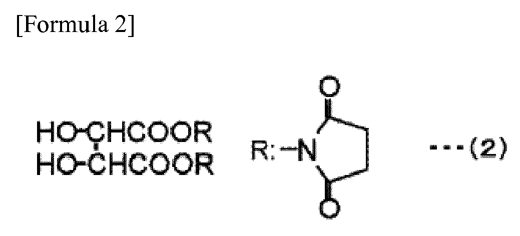

### <Tissue adhesion with use of tissue adhesive film of this embodiment>

Next is a description of the tissue adhesion with use of the tissue adhesive film of this embodiment.

First, the tissue adhesive film is pasted to a surface of a tissue and left standing. By so doing, the tissue adhesive film is adhered to the tissue.

The leaving time is necessary for the tissue adhesive film to absorb the moisture in the tissue enough to achieve cross-linking. The time is appropriately set according to the proportion of the constituent materials in the tissue adhesive film. For example, the time can be set to about 10 minutes. In addition, at this time, the tissue adhesive film and the tissue may be heated as long as the temperature is 37°C or lower.

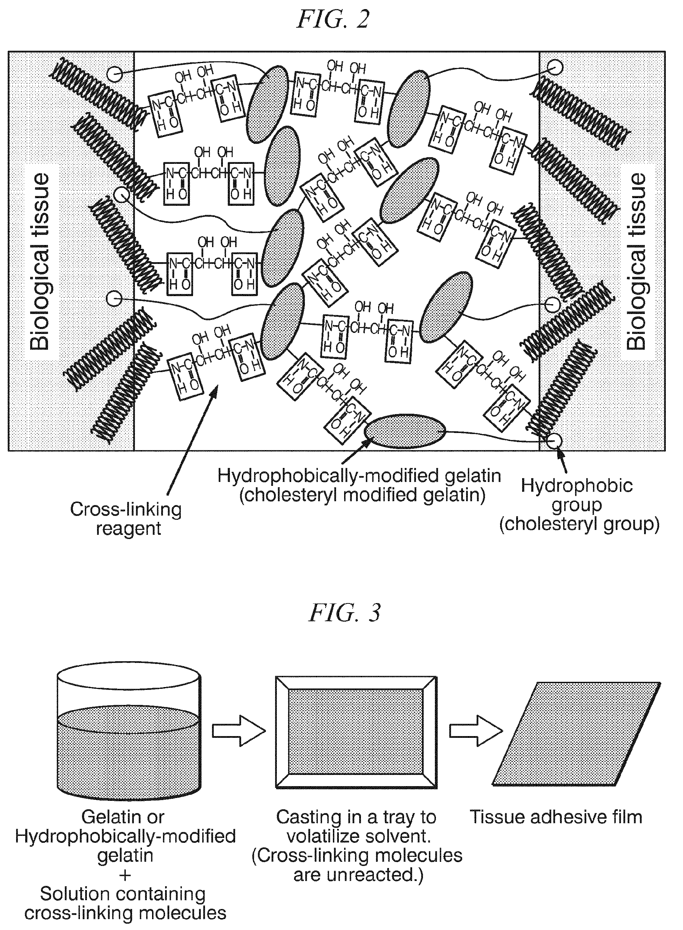

FIG. 2 is a schematic diagram illustrating an example of tissue adhesion with use of the tissue adhesive film serving as an embodiment of the present invention.

As shown in FIG. 2, the tissue adhesive film absorbs moisture in the tissue, by which an active ester of the cross-linking reagent reacts with an amino group of the gelatin or the hydrophobically-modified gelatin. Thereby, an amide bond is formed. Moreover, another active ester group of this cross-linking reagent is replaced with hydrogen of an amino group of another hydrophobically-modified gelatin, by which the active ester group is bound to the amino group. By so doing, two molecules of the hydrophobically-modified gelatin are cross-linked by a single cross-linking reagent.

When this cross-linking reaction occurs in a chain-like manner, a construct in which a plurality of molecules of the hydrophobically-modified gelatin are firmly bound by the cross-linking reagents is formed. By so doing, this construct is made chemically firm.

In other words, it is thought that the chemical reaction represented by the following formula (3) is generated. In the formula (3), -COOR represents an active ester of the cross-linking reagent, and -NH₂ represents an amino group of the gelatin or the hydrophobically-modified gelatin.

### [Formula 3]

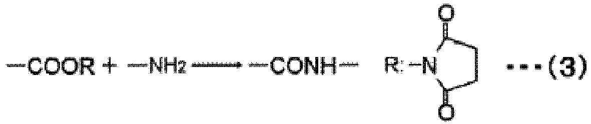

Moreover, as shown in FIG. 2, the hydrophobic groups having a fixed molecular weight and a fixed size are sticking to the tissue through hydrophobic interaction. Therefore, the hydrophobically-modified gelatin is firmly fixed to the tissue. By so doing, the construct is adhered to the surface of the tissue by a physically firm attachment.

In this embodiment, the film is left standing at room temperature until it is solidified after the adhesion. However, the film may be heated so as to accelerate the adhesion rate as long as the temperature is 37°C or lower.

### <Method for producing tissue adhesive film>

Next is a description of a method for producing a tissue adhesive film serving as an embodiment of the present invention.

The method for producing a tissue adhesive film serving as an embodiment of the present invention comprises: a step of synthesizing hydrophobically-modified gelatin, a step of mixing with a cross-linking reagent, and a step of volatilizing a solvent.

### (Synthesis of hydrophobically-modified gelatin)

The step of synthesizing hydrophobically-modified gelatin is a step of adding an organic molecule that has a hydrophobic group, to a solution in which gelatin has been dissolved, under the presence of a triethylamine so as to synthesize hydrophobically-modified gelatin by partially substituting amino groups on the side chain of the gelatin with the hydrophobic group.

The gelatin is selected so that the molecular weight of the hydrophobically-modified gelatin would be 20,000 or higher and 100,000 or lower.

First, an organic molecule that has a hydrophobic group which is reactive with an amino group is mixed with gelatin dissolved in an organic solvent, in the presence of a triethylamine, so as to prepare a mixture solution in a container.

As the organic solvent, dimethylsulfoxide (DMSO) can be used, for example.

The organic molecule can be exemplified by cholesteryl chloroformate represented by the following formula (4).

Next, the mixture solution is heated and stirred under an inert gas atmosphere. For example, this can be done under a nitrogen atmosphere at a heating temperature of 80°C for a stirring time of a day and a night.

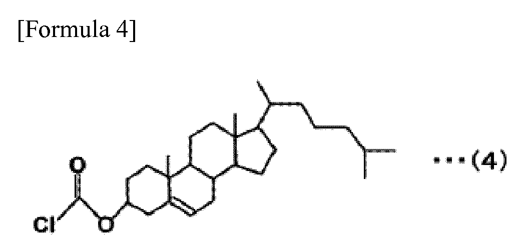

Next, this mixture solution is added dropwise to an ice cold ethanol solvent for the purification of hydrophobically-modified gelatin. Next, this solution is filtrated through a glass filter or the like.

Furthermore, the filtrated matter is washed with an organic solvent. By so doing, impurities in the filtrated matter can be removed so as to improve the purity of the hydrophobically-modified gelatin. Regarding this organic solvent for the washing purpose, ethanol can be used, for example.

From the above-mentioned step, hydrophobically-modified gelatin in which amino groups on the side chain of the gelatin are partially substituted with the hydrophobic group can be synthesized.

### (Step of mixing with cross-linking reagent)

The step of mixing with a cross-linking reagent is a step of dissolving the hydrophobically-modified gelatin or unmodified gelatin in a fluorine based solvent, and thereafter mixing it with a cross-linking reagent which has two or more active ester groups or anhydrides within a single molecule.

First, hydrophobically-modified gelatin is dissolved in a fluorine based solvent. The fluorine based solvent can be exemplified by hexafluoroisopropanol. Since the boiling point is 56°C, volatilization can be readily done by a step that will be described later.

Next, the fluorine based solvent dissolved with the hydrophobically-modified gelatin or the unmodified gelatin is mixed with a cross-linking reagent which has two or more active ester groups or anhydrides within a single molecule. At this time, the cross-linking reagent has not caused a cross-linking reaction with the hydrophobically-modified gelatin.

### (Step of casting hydrophobically-modified gelatin solution)

Next, the mixture solution is charged in a container such as a tray, a petri dish, or the like, and then left standing at room temperature to 35°C. By so doing, the fluorine based solvent is volatilized.

From the above-mentioned step, a tissue adhesive film comprising an integration of gelatin or hydrophobically-modified gelatin and cross-linking reagents, wherein the gelatin has a molecular weight of 50,000 or higher and 100,000 or lower and is provided with an amino group and a hydrophobic group on the side chain thereof, and the cross-linking reagent has two or more active ester groups or anhydrides within a single molecule, can be readily produced. At this time, the cross-linking reagent does not cause a reaction with the hydrophobically-modified gelatin.

In the tissue adhesive film serving as an embodiment of the present invention, unmodified gelatin (referred to as original gelatin) can be additionally mixed therein in order to adjust the proportion of the hydrophobically-modified gelatin in the tissue adhesive film to an optimum value so as to much improve the adhesive strength.

In the tissue adhesive film serving as an embodiment of the present invention, the structure is such that the tissue adhesive film comprises an integration of gelatin and cross-linking reagents, wherein the gelatin is provided with an amino group on the side chain thereof and has a molecular weight of 50,000 or higher and 100,000 or lower, and the cross-linking reagent has two or more active ester groups or anhydrides within a single molecule. Therefore, the amino groups of the hydrophobically-modified gelatin can be mutually cross-linked by the active ester groups or the acid anhydride of the cross-linking reagent. Thus, a chemically firm attachment can be created, by which the adhesive strength can be enhanced. In addition, the hydrophobically-modified gelatin can be readily decomposed by an enzyme (collagenase) in the course of wound healing. Therefore, inflammation reaction, calcification, and the like can be suppressed.

In the tissue adhesive film of the present invention, the structure is such that a part or all of the gelatin is hydrophobically-modified gelatin which is provided with a hydrophobic group on the side chain thereof. Thus, a physically firm attachment can be created by having the hydrophobic group piercing (anchored) into the tissue, by which the adhesive strength can be enhanced.

In the tissue adhesive film serving as an embodiment of the present invention, the structure is such that the hydrophobically-modified gelatin is a polymer in which two or more amino acids are linearly linked and the amino groups of Lys, contained as the above-mentioned amino acids, are partially substituted with the hydrophobic group. Since Lys-derived amino groups are held, the amino groups existing in the gelatin or the hydrophobically-modified gelatin can be mutually cross-linked by the active ester groups of the cross-linking reagent. Thus, a chemically firm attachment can be created, by which the adhesive strength can be enhanced. In addition, the hydrophobically-modified gelatin can be readily decomposed by an enzyme (collagenase) in the course of wound healing, by which the biocompatibility can be enhanced.

In the tissue adhesive film serving as an embodiment of the present invention, the structure is such that the hydrophobic group is any one type or a combination of two or more types of a cholesteryl group, an oleyl group, an isostearyl group, a stearyl group, an isopalmityl group, a myristyl group, a lauryl group, a capric group, a pelargol group, a caprylic group, a caproyl group, an α-linolenyl group, a stearidonyl group, an eicosapentaenoyl group, and a docosahexaenyl group. Thus, a physically firm attachment can be created by having the hydrophobic group piercing (anchored) into the tissue, by which the adhesive strength can be enhanced.

In the tissue adhesive film serving as an embodiment of the present invention, the structure is such that the gelatin is any one type or a combination of two or more types of gelatin derived from human, porcine, bovine, and fish species, or genetically modified gelatin. Thus, the attachment of the tissue adhesive film can be made chemically firm by binding the anchored hydrophobically-modified gelatin and the unmodified gelatin.

In the tissue adhesive film serving as an embodiment of the present invention, the structure is such that the cross-linking reagent is any one type or a combination of two or more types selected from the group consisting of tartaric acid, citric acid, malic acid, glutamic acid, aspartic acid, oxalacetic acid, cis-aconitic acid, 2-ketoglutaric acid, polytartaric acid, polycitric acid, polymalic acid, polyglutamic acid, and polyaspartic acid, wherein it has two or more active ester groups are held within a single molecule of the above-mentioned molecules, or it has acid anhydrides. Thus, the amino groups of the gelatin or the hydrophobically-modified gelatin can be mutually cross-linked by the active ester groups or the acid anhydride of the cross-linking reagent, by which the attachment of the tissue adhesive film can be made chemically firm.

The method for producing a tissue adhesive film serving as an embodiment of the present invention comprises: a step of dispersing gelatin or hydrophobically-modified gelatin in a fluorine based solvent, and thereafter mixing it with a cross-linking reagent which has two or more active ester groups or anhydrides within a single molecule; and a step of volatilizing the fluorine based solvent. Therefore, a tissue adhesive film having high adhesive strength and high biocompatibility can be readily produced.

The method for producing a tissue adhesive film serving as an embodiment of the present invention comprises a step of adding an organic molecule that has a hydrophobic group, to a solution in which gelatin has been dissolved, under the presence of an amine, so as to synthesize hydrophobically-modified gelatin by partially substituting amino groups on the side chain of the gelatin with the hydrophobic group Therefore, a tissue adhesive film having high adhesive strength and high biocompatibility can be readily produced.

In the method for producing a tissue adhesive film serving as an embodiment of the present invention, the structure is such that the fluorine based solvent is hexafluoroisopropanol. Thus, the tissue adhesive film can be readily produced by volatilizing the fluorine based solvent.

The tissue adhesive film and the method for producing the same serving as the embodiments of the present invention are not to be limited to the above-mentioned embodiments, and various modifications can be made and executed within the scope of the technical idea of the present invention. Specific examples of these embodiments are shown in the following Examples. However, the present invention is not to be limited to these Examples.

### Examples

### (Preparation of tissue adhesive film made of gelatin)

### (Example 1)

First, an alkali-treated gelatin was prepared.

The alkali-treated gelatin means gelatin in which asparagine and glutamine existing in the gelatin have been converted into aspartic acid and glutamic acid by deamidation.

10.52 g of the alkali-treated gelatin was dissolved in 70 mL of hexafluoroisopropanol to obtain a 15% alkali-treated gelatin solution.

2.5 mL of the obtained solution was mixed with 96.6 mg of trisuccinimidyl citrate (TSC) serving as the cross-linking reagents, and the mixture was dissolved. By so doing, a solution for producing the tissue adhesive film of Example 1 was produced. At this time, the preparation was conducted so that the mol concentration of TSC in the solution for producing the tissue adhesive film would be 80 mM.

Next, the solution was placed in a Teflon (registered trademark) tray (bottom size of 8 cm x 5 cm) and left standing on a hot substrate at 35°C in a draft to volatilize the hexafluoroisopropanol solution. The tray was left standing for 3 days, and then was vacuum-dried over night. By so doing, a tissue adhesive film was produced.

### (Evaluation of formation of tissue adhesive film)

The obtained tissue adhesive film was punched with a hole punch having a diameter of 8 mm and put in a 15 mL plastic test tube. Then, 10 mL of ultrapure water at 4°C or 37°C was poured therein. By so doing, the progression of the cross-linking reaction and the film formation were evaluated.

### (Evaluation of adhesiveness of tissue adhesive film)

The obtained tissue adhesive film (diameter of 8 mm) was left still in a rat liver for 5 minutes, and the tissue adhesive film was pulled with tweezers. By so doing, the adhesiveness was qualitatively evaluated.

A tissue adhesive film having been prepared not using the cross-linking reagent but using the gelatin alone was used as a Comparative Example.

### (Evaluation of biocompatibility of tissue adhesive film)

The obtained tissue adhesive film (diameter of 8 mm) was left still in a rat liver for 5 minutes, and then the abdomen was closed. The condition after 7 days was evaluated by eye.

A tissue adhesive film having been prepared not using the cross-linking reagent but using the gelatin alone was used as a Comparative Example.

### (Evaluation of sealing of tissue adhesive film)

First, a part of the tissue adhesive film was cut out, and pasted to close a hole (diameter of 1 mm) made in a rat lung, and left for 7 minutes.

After 5 minutes, the tissue adhesive film was confirmed to be solidified. Moreover, no presence of any other hole in the rat lung was confirmed.

Next, a syringe was inserted in the rat lung and air was injected therein.

The maximum pressure at the time when air started to leak from the rat lung was measured.

Regarding the tissue adhesive film prepared in Example 1, the cross-linking reaction made progress by the addition of 4°C or 37°C water. This resulted in the film formation.

Moreover, the adhesiveness to the rat liver was observed. After 7 days, the degradability was observed. The maximum pressure at the time when air started to leak from the rat lung was 916 mmHg.

### (Example 2)

The tissue adhesive film of Example 2 was produced in the same manner as that of Example 1, except for that the TSC concentration in the tissue adhesive film was set to 40 mM (addition of 49.9 mg).

Regarding the tissue adhesive film prepared in Example 2, the cross-linking reaction made progress by the addition of 4°C or 37°C water. This resulted in the film formation.

Moreover, the adhesiveness to the rat liver was observed.

### (Example 3)

The tissue adhesive film of Example 3 was produced in the same manner as that of Example 1, except for that the TSC concentration in the tissue adhesive film was set to 20 mM (addition of 24.0 mg).

Regarding the tissue adhesive film prepared in Example 3, the cross-linking reaction made progress by the addition of 4°C or 37°C water. This resulted in the film formation.

### (Example 4)

The tissue adhesive film of Example 4 was produced in the same manner as that of Example 1, except for that the TSC concentration in the tissue adhesive film was set to 10 mM (addition of 12.74 mg).

Regarding the tissue adhesive film prepared in Example 4, the cross-linking reaction made progress by the addition of 4°C or 37°C water. This resulted in the film formation.

### (Example 5)

The tissue adhesive film of Example 5 was produced in the same manner as that of Example 1, except for that the TSC concentration in the tissue adhesive film was set to 5 mM (addition of 6.45 mg).

Regarding the tissue adhesive film prepared in Example 5, the cross-linking reaction made progress by the addition of 4°C water. This resulted in the film formation. In the case of 37°C, a swollen film was formed.

### (Example 6)

The tissue adhesive film of Example 6 was produced in the same manner as that of Example 1, except for that disuccinimidyl malate (DSM) was used instead of TSC in the tissue adhesive film and the DSM concentration was set to be 80 mM (addition of 65.59 mg).

Regarding the tissue adhesive film prepared in Example 6, the cross-linking reaction made progress by the addition of 4°C water. This resulted in the film formation. In the case of 37°C, there was no film formation. Moreover, the adhesiveness to the rat liver was observed. After 7 days, the degradability was observed.

### (Example 7)

The tissue adhesive film of Example 7 was produced in the same manner as that of Example 1, except for that the DSM concentration in the tissue adhesive film was set to 40 mM (addition of 32.2 mg).

Regarding the tissue adhesive film prepared in Example 7, the cross-linking reaction made progress by the addition of 4°C or 37°C water. This resulted in the film formation.

### (Example 8)

The tissue adhesive film of Example 8 was produced in the same manner as that of Example 1, except for that the DSM concentration in the tissue adhesive film was set to 20 mM (addition of 15.88 mg).

Regarding the tissue adhesive film prepared in Example 8, the cross-linking reaction made progress by the addition of 4°C water. This resulted in the film formation. In the case of 37°C, a swollen film was formed.

### (Example 9)

The tissue adhesive film of Example 9 was produced in the same manner as that of Example 1, except for that the DSM concentration in the tissue adhesive film was set to 10 mM (addition of 8.79 mg).

Regarding the tissue adhesive film prepared in Example 9, the cross-linking reaction made progress by the addition of 4°C water. This resulted in the film formation. In the case of 37°C, there was no film formation.

### (Example 10)

The tissue adhesive film of Example 10 was produced in the same manner as that of Example 1, except for that the DSM concentration in the tissue adhesive film was set to 5 mM (addition of 4.38 mg).

Regarding the tissue adhesive film prepared in Example 10, the cross-linking reaction made progress by the addition of 4°C water. This resulted in the film formation. In the case of 37°C, there was no film formation.

### (Example 11)

The tissue adhesive film of Example 11 was produced in the same manner as that of Example 1, except for that disuccinimidyl tartrate (DST) was used instead of TSC in the tissue adhesive film and the DST concentration was set to be 80 mM (addition of 69.64 mg).

Regarding the tissue adhesive film prepared in Example 11, the cross-linking reaction made progress by the addition of 4°C water. This resulted in the film formation. In the case of 37°C, there was no film formation. Moreover, the adhesiveness to the rat liver was observed. After 7 days, the degradability was observed.

### (Example 12)

The tissue adhesive film of Example 12 was produced in the same manner as that of Example 1, except for that the DST concentration in the tissue adhesive film was set to 40 mM (addition of 35.35 mg). Regarding the tissue adhesive film prepared in Example 12, the cross-linking reaction made progress by the addition of 4°C or 37°C water. This resulted in the film formation.

### (Example 13)

The tissue adhesive film of Example 13 was produced in the same manner as that of Example 1, except for that the DST concentration in the tissue adhesive film was set to 20 mM (addition of 17.79 mg). Regarding the tissue adhesive film prepared in Example 13, the cross-linking reaction made progress by the addition of 4°C water. This resulted in the film formation. In the case of 37°C, a swollen film was formed.

### (Example 14)

The tissue adhesive film of Example 14 was produced in the same manner as that of Example 1, except for that the DST concentration in the tissue adhesive film was set to 10 mM (addition of 8.52 mg).

Regarding the tissue adhesive film prepared in Example 14, the cross-linking reaction made progress by the addition of 4°C water. This resulted in the film formation. In the case of 37°C, there was no film formation.

### (Example 15)

The tissue adhesive film of Example 15 was produced in the same manner as that of Example 1, except for that the DST concentration in the tissue adhesive film was set to 5 mM (addition of 4.01 mg).

Regarding the tissue adhesive film prepared in Example 15, the cross-linking reaction made progress by the addition of 4°C water. This resulted in the film formation. In the case of 37°C, there was no film formation.

### (Example 16)

### (Synthesis of hydrophobically-modified gelatin (cholesteryl modified gelatin))

First, 1 g of gelatin having a molecular weight of 100,000 (derived from porcine skin, a product of Nitta Gelatin Inc.) was dissolved by stirring in 9.9 mL of DMSO, and kept at 80°C under a nitrogen atmosphere. 0.1 mL of triethylamine was added thereto. Cholesteryl chloroformate in a powdery form was added thereto at 10% of the amino groups in the gelatin, and dissolved by stirring at 80°C. This was sealed in a calcium chloride tube, and allowed to react by stirring for a day and a night.

The reaction solution was added dropwise to a 30 mL of ice cold ethanol so as to stop the reaction. The reaction product was precipitated, and then was filtrated through a glass filter. Thereafter, this was washed with 10 mL of ethanol three times. The residue in the glass filter was washed with 10 mL of ethyl acetate three times and then vacuum-dried at 60°C for a day and a night. By so doing, cholesteryl modified gelatin was obtained. The introduction of the cholesteryl group was confirmed by proton nuclear magnetic resonance (1H-NMR), which showed the peak of C18 proton around 0.67 ppm.

### (Preparation of tissue adhesive film)

0.3 g of the obtained cholesteryl modified gelatin was dissolved in 2 mL of hexafluoroisopropanol to obtain a 15% cholesteryl modified gelatin solution. 0.5 mL of the obtained solution was mixed with 19.32 mg of trisuccinimidyl citrate (TSC) serving as the cross-linking reagents and the mixture was dissolved. By so doing, a solution for producing the tissue adhesive film of Example 16 was produced. At this time, the preparation was conducted so that the mol concentration of TSC in the solution for producing the tissue adhesive film would be 80 mM. The solution was placed in a Teflon (registered trademark) tray (bottom size of 8 cm x 5 cm) and left standing on a hot substrate at 35°C in a draft to volatilize the hexafluoroisopropanol solution. The tray was left standing for 3 days, and then was vacuum-dried over night. By so doing, a tissue adhesive film was produced.

The obtained tissue adhesive film was punched with a hole punch having a diameter of 8 mm and put in a 15 mL plastic test tube. Then, 10 mL of ultrapure water at 4°C was poured therein. By so doing, the progression of the cross-linking reaction and the film formation were evaluated.

Regarding the tissue adhesive film prepared in Example 16, the cross-linking reaction made progress by the addition of 4°C water. This resulted in the film formation.

### (Example 17)

The tissue adhesive film of Example 17 was produced in the same manner as that of Example 16, except for that the TSC concentration in the tissue adhesive film was set to 40 mM (addition of 9.98 mg).

Regarding the tissue adhesive film prepared in Example 17, the cross-linking reaction made progress by the addition of 4°C water. This resulted in the film formation.

### (Example 18)

The tissue adhesive film of Example 18 was produced in the same manner as that of Example 16, except for that the TSC concentration in the tissue adhesive film was set to 20 mM (addition of 4.8 mg).

Regarding the tissue adhesive film prepared in Example 18, the cross-linking reaction made progress by the addition of 4°C water. This resulted in the film formation.

### (Example 19)

The tissue adhesive film of Example 19 was produced in the same manner as that of Example 16, except for that cholesteryl chloroformate in a powdery form was added at 50% of the amino groups in the gelatin in the tissue adhesive film, and the TSC concentration was set to 80 mM (addition of 19.32 mg).

Regarding the tissue adhesive film prepared in Example 19, the cross-linking reaction made progress by the addition of 4°C water. This resulted in the film formation.

### (Example 20)

The tissue adhesive film of Example 20 was produced in the same manner as that of Example 16, except for that cholesteryl chloroformate in a powdery form was added at 50% of the amino groups in the gelatin in the tissue adhesive film, and the TSC concentration was set to 20 mM (addition of 4.8 mg).

Regarding the tissue adhesive film prepared in Example 20, the cross-linking reaction made progress by the addition of 4°C water. This resulted in the film formation.

### (Example 21)

The tissue adhesive film of Example 21 was produced in the same manner as that of Example 16, except for that cholesteryl chloroformate in a powdery form was added at 50% of the amino groups in the gelatin in the tissue adhesive film, and the TSC concentration was set to 5 mM (addition of 0.6 mg).

Regarding the tissue adhesive film prepared in Example 21, the cross-linking reaction made progress by the addition of 4°C water. This resulted in the film formation.

### (Comparative Example 1)

In the case where gelatin having a molecular weight of 100,000 was used without including the cross-linking reagent, the film was swollen at 4°C and the film was not formed but dissolved at 37°C.

### (Comparative Example 2)

The tissue adhesive film of Comparative Example 2 was produced in the same manner as that of Example 16, except for that gelatin having a molecular weight of 20,000 was used.

The tissue adhesive film of Comparative Example 2 was too fragile to be formed as a film.

### (Comparative Example 3)

The tissue adhesive film of Comparative Example 3 was produced in the same manner as that of Example 16, except for that gelatin having a molecular weight of 50,000 was used.

The tissue adhesive film of Comparative Example 3 was too fragile to be formed as a film.

### INDUSTRIAL APPLICABILITY

The tissue adhesive film and the method for producing the same of the present invention relate to a tissue adhesive film having high adhesive strength and high biocompatibility and a method for producing the same. Therefore, they are industrially applicable in the industrial fields where a tissue adhesive, a tissue sealant, a hemostatic agent, and the like are required, and such other fields.

## Claims

1. A tissue adhesive film comprising an integration of:
gelatin which is provided with an amino group on the side chain and has a molecular weight of 50,000 or higher and 100,000 or lower, and a part or all of which is hydrophobically-modified gelatin which is provided with a hydrophobic group on the side chain thereof, wherein said hydrophobic group is any one type or a combination of two or more types selected from the group consisting of a cholesteryl group, an oleyl group, an isostearyl group, a stearyl group, an isopalmityl group, a myristyl group, a lauryl group, a capric group, a pelargol group, a caprylic group, a caprol group, an α-linolenyl group, a stearidonyl group, an eicosapentaenoyl group, and a docosahexaenyl group; and
cross-linking reagents which have two or more active ester groups or anhydrides within a single molecule.

2. A tissue adhesive film according to claim 1, wherein said hydrophobically-modified gelatin is a polymer in which two or more amino acids are linearly linked and Lys residues in the polymer are partially substituted with said hydrophobic group.

3. A tissue adhesive film according to claim 1 or claim 2, wherein said gelatin is any one type or a combination of two or more types selected from the group consisting of natural gelatin derived from human, porcine, bovine, or fish species, or genetically-modified gelatin thereof.

4. A tissue adhesive film according to any one of claims 1, 2 and 3, wherein said cross-linking reagent is any one type or a combination of two or more types selected from the group consisting of tartaric acid, citric acid, malic acid, glutamic acid, aspartic acid, oxalacetic acid, cis-aconitic acid, 2-ketoglutaric acid, polytartaric acid, polycitric acid, polymalic acid, polyglutamic acid, and polyaspartic acid.

5. A method for producing a tissue adhesive film comprising:
(1) a step of dispersing hydrophobically-modified gelatin in a fluorine based solvent, and thereafter mixing it with a cross-linking reagent which has two or more active ester groups or anhydrides within a single molecule; and
(2) a step of volatilizing said fluorine based solvent, wherein a part or all of the hydrophobically-modified gelatin is provided with a hydrophobic group on the side chain thereof, and said hydrophobic group is any one type or a combination of two or more types selected from the group consisting of a cholesteryl group, an oleyl group, an isostearyl group, a stearyl group, an isopalmityl group, a myristyl group, a lauryl group, a capric group, a pelargol group, a caprylic group, a caprol group, an α-linolenyl group, a stearidonyl group, an eicosapentaenoyl group, and a docosahexaenyl group.

6. A method for producing a tissue adhesive film according to claim 5, wherein said fluorine based solvent is hexafluoroisopropanol.

## Patentansprüche

1. Gewebehaftfilm, umfassend eine Integration von:
Gelatine, die mit einer Aminogruppe an der Seitenkette versehen ist und ein Molekulargewicht von 50.000 oder mehr und 100.000 oder weniger hat, und ein Teil der oder alle Gelatine eine hydrophob modifizierte Gelatine ist, die mit einer hydrophoben Gruppe an deren Seitenkette versehen ist, wobei die hydrophobe Gruppe ein beliebiger Typ oder eine Kombination von zwei oder mehr Typen ist, ausgewählt aus der Gruppe bestehend aus einer Cholesteryl-Gruppe, einer Oleyl-Gruppe, einer Isostearyl-Gruppe, einer Stearyl-Gruppe, einer Isopalmityl-Gruppe, einer Myristyl-Gruppe, einer Lauryl-Gruppe, einer Decan-Gruppe, einer Pelargol-Gruppe, einer Capryl-Gruppe, einer Caprol-Gruppe, einer α-Linolenyl-Gruppe, einer Stearidonyl-Gruppe, einer Eicosapentaenoyl-Gruppe und einer Docosahexaenyl-Gruppe; und
Vernetzungsmitteln, die zwei oder mehr aktive Estergruppen oder Anhydride innerhalb eines einzigen Moleküls haben.

2. Gewebehaftfilm gemäß Anspruch 1, wobei die hydrophob modifizierte Gelatine ein Polymer ist, bei dem zwei oder mehr Aminosäuren linear verknüpft sind und Lysinreste in dem Polymer teilweise durch die hydrophobe Gruppe ersetzt sind.

3. Gewebehaftfilm gemäß Anspruch 1 oder Anspruch 2, wobei die Gelatine ein beliebiger Typ oder eine Kombination aus zwei oder mehr Typen ist, ausgewählt aus der Gruppe bestehend aus natürlicher Gelatine, die sich von Mensch-, Schwein-, Rind- oder Fisch-Spezies oder deren genetisch modifizierter Gelatine ableitet.

4. Gewebehaftfilm gemäß einem der Ansprüche 1, 2 und 3, wobei das Vernetzungsmittel ein beliebiger Typ oder eine Kombination aus zwei oder mehr Typen ist, ausgewählt aus der Gruppe bestehend aus Weinsäure, Zitronensäure, Apfelsäure, Glutaminsäure, Asparaginsäure, Oxalessigsäure, cis-Aconitsäure, 2-Ketoglutarsäure, Polyweinsäure, Polyzitronensäure, Polyapfelsäure, Polyglutaminsäure und Polyasparaginsäure.

5. Verfahren zum Herstellen eines Gewebehaftfilms, umfassend:
1) einen Schritt des Dispergierens hydrophob modifizierter Gelatine in einem auf Fluor basierenden Lösungsmittel, und danach Vermischung dieses mit einem Vernetzungsmittel, das zwei oder mehr aktive Estergruppen oder Anhydride innerhalb eines einzigen Moleküls hat; und
2) einen Schritt des Verdampfens des auf Fluor basierenden Lösungsmittels, wobei ein Teil der oder alle hydrophob modifizierte Gelatine mit einer hydrophoben Gruppe an deren Seitenkette versehen ist und die hydrophobe Gruppe ein beliebiger Typ oder eine Kombination von zwei oder mehr Typen ist, ausgewählt aus der Gruppe bestehend aus einer Cholesteryl-Gruppe, einer Oleyl-Gruppe, einer Isostearyl-Gruppe, einer Stearyl-Gruppe, einer Isopalmityl-Gruppe, einer Myristyl-Gruppe, einer Lauryl-Gruppe, einer Decan-Gruppe, einer Pelargol-Gruppe, einer Capryl-Gruppe, einer Caprol-Gruppe, einer α-Linolenyl-Gruppe, einer Stearidonyl-Gruppe, einer Eicosapentaenoyl-Gruppe und einer Docosahexaenyl-Gruppe.

6. Verfahren zum Herstellen eines Gewebehaftfilms gemäß Anspruch 5, wobei das auf Fluor basierende Lösungsmittel Hexafluorisopropanol ist.

## Revendications

1. Film adhésif tissulaire, comprenant, à l'état intégré :
- une gélatine portant un groupe amino sur une chaîne latérale et présentant un poids moléculaire d'au moins 50 000 et d'au plus 100 000, et dont tout ou partie est une gélatine modifiée hydrophobe portant un groupe hydrophobe sur une chaîne latérale, lequel groupe hydrophobe est d'un seul type ou d'une combinaison de deux types ou plus, choisi(s) dans l'ensemble formé par les groupes cholestéryle, oléyle, isostéaryle, stéaryle, isopalmityle, myristyle, lauryle, capryle, pélargyle, caprylyle, caproyle, α-linolényle, stéaridonyle, eicosapentaénoyle et docosahexaénoyle ;
- et des réactifs de réticulation comportant deux groupes de type ester actif ou anhydride dans une seule molécule.

2. Film adhésif tissulaire conforme à la revendication 1, dans lequel ladite gélatine modifiée hydrophobe est un polymère dans lequel deux acides aminés ou plus sont reliés linéairement et une partie des résidus de lysine présents dans le polymère portent ledit groupe hydrophobe en tant que substituant.

3. Film adhésif tissulaire conforme à la revendication 1 ou 2, dans lequel ladite gélatine est d'un seul type ou d'une combinaison de deux types ou plus, choisi(s) dans l'ensemble formé par les gélatines naturelles dérivées des espèces humaine, porcines, bovines ou de poisson, ou une variante génétiquement modifiée d'une telle gélatine.

4. Film adhésif tissulaire conforme à l'une des revendications 1, 2 et 3, dans lequel ledit réactif de réticulation est d'un seul type ou d'une combinaison de deux types ou plus, choisi(s) dans l'ensemble formé par les acides tartrique, citrique, malique, glutamique, aspartique, oxalacétique, cis-aconitique et 2-céto-glutarique et les poly(acide tartrique), poly(acide citrique), poly(acide malique), poly(acide glutamique et poly(acide aspartique).

5. Procédé de production d'un film adhésif tissulaire, comportant les étapes suivantes :
1) disperser une gélatine modifiée hydrophobe dans un solvant fluoré et y mélanger ensuite un réactif de réticulation comportant deux groupes de type ester actif ou anhydride dans une seule molécule,
2) et volatiliser ledit solvant fluoré,
et dans lequel tout ou partie de la gélatine modifiée hydrophobe porte un groupe hydrophobe sur une chaîne latérale, lequel groupe hydrophobe est d'un seul type ou d'une combinaison de deux types ou plus, choisi(s) dans l'ensemble formé par les groupes cholestéryle, oléyle, isostéaryle, stéaryle, isopalmityle, myristyle, lauryle, capryle, pélargyle, caprylyle, caproyle, α-linolényle, stéaridonyle, eicosapentaénoyle et docosahexaénoyle.

6. Procédé de production d'un film adhésif tissulaire, conforme à la revendication 5, dans lequel ledit solvant fluoré est de l'hexafluoro-isopropanol.
